(19) Europäisches Patentamt
European Patent Office
Office européen des brevets

(11) Veröffentlichungsnummer : **0 167 097**
**B1**

(12) **EUROPÄISCHE PATENTSCHRIFT**

(45) Veröffentlichungstag der Patentschrift : **30.09.87**

(21) Anmeldenummer : **85107886.5**

(22) Anmeldetag : **25.06.85**

(51) Int. Cl.[4] : **C 07 C 17/00**, C 07 C 51/00, C 07 C 45/65, C 07 C 57/54, C 07 C 49/687, C 07 C 49/80, C 25 B 3/04, C 07 C 67/27, C 07 C 29/00

(54) **Verfahren zur Herstellung von Chlorolefinen.**

(30) Priorität : **28.06.84 DE 3423762**

(43) Veröffentlichungstag der Anmeldung : **08.01.86 Patentblatt 86/02**

(45) Bekanntmachung des Hinweises auf die Patenterteilung : **30.09.87 Patentblatt 87/40**

(84) Benannte Vertragsstaaten : **BE CH DE FR GB IT LI NL**

(56) Entgegenhaltungen :
**DD-A- 151 187**
**DE-A- 2 657 148**
**DE-A- 3 224 162**
**US-A- 4 061 548**
**ANGWANDTE CHEMIE, Band 92, 1980, Weinheim, J. WELLMANN et al. "Chrom(II)-katalysierte elektrochemische Enthalogenierung von beta-Hydroxyhalogeniden - ein bequemer Zugang zu Des-oxynucleoxiden", Seiten 47,48**
**CHOLLECTION OF CZECHOSLOVAK CHEMICAL COMMUNICATIONS, Band 24, 1959, J. FARKAS et al. "Relation between chemical structure and insecticidal activity in pyrethroid compounds", Seiten 2230-2236**
**Tetrahedron, vol. 20 (1964), pp. 1005-1015**
**JACS 85 (1963), p. 2768**
**JACS 88 (1966), pp. 4447, 4448**

(73) Patentinhaber : **BASF Aktiengesellschaft**
**Carl-Bosch-Strasse 38**
**D-6700 Ludwigshafen (DE)**

(72) Erfinder : **Steckhan, Eberhard, Prof. Dr.**
**Jungholzweg 26**
**D-5309 Meckenheim (DE)**
Erfinder : **Wolf, Reinhard**
**Siebengebirgstrasse 144**
**D-5330 Koenigswinter-Thomasberg (DE)**
Erfinder : **Puetter, Hermann, Dr.**
**Haardter Strasse 1**
**D-6730 Neustadt (DE)**

EP 0 167 097 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Diese Erfindung betrifft ein Verfahren zur Herstellung von Chlorolefinen durch Behandlung von Trichlormethylverbindungen mit Chrom-II-Salzen in wäßrigem Medium sowie neue Chlorolefine.

Chlorolefine sind gesuchte Zwischenprodukte, die z. B. für die Herstellung von Pyrethroiden benötigt werden.

Man gewinnt sie z. B. durch Reduktion von 1,1,1-Trichlormethyl-2-hydroxiverbindungen. Nach einem in der DE-OS 26 57 148 beschriebenen Verfahren werden Halogenalkohole dieser Art elektrochemisch zu den entsprechenden halogenierten Dienverbindungen dehydrochloriert. Man kann die Halogenalkohole auch acetylieren und durch Behandlung mit Zinkstaub in die Chlorolefine überführen (Coll. Czech. Chem. Common. 1959, 24, 2230-36). Nach den Angaben der DE-A-3 224 162 werden 1,1,1-Trihalogenmethyl-2-acyloxiverbindungen durch Behandlung mit Zink, Aluminium, Zinn, Eisen oder Magnesium in mineralsaurem Medium zu den entsprechenden Dihalogenolefinen umgesetzt. Ein weiteres elektrochemisches Verfahren, bei dem man ß-Trihalogenmethyl-ß-propiolactone kathodisch zu Dihalogenvinylessigsäuren reduziert, ist aus der DD-PS 151 187 bekannt.

Diese Verfahren haben verschiedene Nachteile. Bei der Reduktion mit Zink hat man es mit einem Zwangsanfall von umweltbelastenden Zink-Salzen zu tun. Außerdem ist es meist notwendig, die Ausgangsstufe in einem zusätzlichen Verfahrensschritt erst zu acetylieren. Die elektrochemische Reduktion ist zwar vorteilhafter, sie leidet jedoch wie alle bekannten Verfahren der direkten Elektroreduktion daran, daß die Reaktion in homogener Lösung durchgeführt werden muß. Man ist also auf Lösungsmittel, wie Essigsäure, angewiesen. Da der Einsatz relativ großer Mengen an organischem Lösungsmittel auch im Anodenraum unumgänglich ist, lassen sich Verluste nicht vermeiden. Außerdem wird auf diese Weise die Lebenszeit der Anoden durch Korrosion herabgesetzt. Schließlich ist es bei der elektrochemischen Reduktion von Trichlormethylverbindungen schwierig, über die Stufe der Dichlorverbindung hinauszukommen. Monochlorolefine werden meist nicht gebildet. Aus Angew. Chemie 92 (1980) S. 47 ist bekannt, daß man bei der Umsetzung von β-Hydroxihalogeniden mit Chrom-II-Salzen sowohl die enthalogenierten Alkohole als auch Olefine erhält. Über Trichlormethylverbindungen oder Verhalten gegenüber Chrom-II-Salzen werden keine Angaben gemacht.

Es wurde nun gefunden, daß man Chlorolefine der allgemeinen Formel

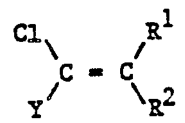

(I)

in der Y ein Chlor- oder Wasserstoffatom, $R^1$ ein Wasserstoffatom, einen Kohlenwasserstoffrest oder einen Rest, der zusammen mit $R^2$ Glied eines Kohlenwasserstoffringes ist, $R^2$ eine Carboxyl-, Carbalkoxy-, Carboxamid-, Nitril- oder Acylgruppe, einen Kohlenwasserstoffrest, der Halogenatome, Hydroxyl-, Keto-, Carboxyl-, Carbalkoxy-, Carboxamid-, Nitril- oder Acylgruppen enthalten kann, oder einen Rest, der zusammen mit $R^1$ Glied eines Kohlenwasserstoffringes ist, bedeuten, vorteilhaft dadurch herstellen kann, daß man Trichlormethylverbindungen der Formel

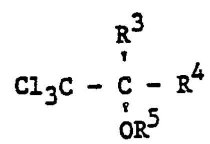

(II)

in der $R^3$ ein Wasserstoffatom, einen Kohlenwasserstoffrest oder einen Rest, der zusammen mit $R^4$ Glied eines Kohlenwasserstoffringes ist, $R^4$ eine Carboxyl-, Carbalkoxy-, Carboxamid-, Nitril- oder Acylgruppe, einen Kohlenwasserstoffrest, der Halogenatome, Hydroxyl-, Keto-, Carboxyl-, Carbalkoxy-, Carboxamid-, Nitril- oder Acylgruppen enthalten kann, oder einen Rest, der zusammen mit $R^3$ Glied eines Kohlenwasserstoffringes ist, oder einen Rest, der zusammen mit $—OR^5$ Glied eines cyclischen Ethers oder eines Lactonringe ist, $R^5$ ein Wasserstoffatom, einen Alkyl- oder Acylrest oder einen Rest, der zusammen mit $R^4$ Glied eines cyclischen Ethers oder eines Lactonringes ist, bedeuten, in mäßrigem Medium mit Chrom-II-Salzen behandelt.

In den als Ausgangsverbindungen zu verwendenden Trichlormethylverbindungen der Formel IV steht der Kohlenwasserstoffrest $R^3$ z. B. für einen Alkyl-, Cycloalkyl-, Alkenyl-, Alkinyl- oder Benzylrest, vorzugsweise für einen Alkylrest mit 1 bis 3 C-Atomen, Als Kohlenwasserstoffringe, an denen die Glieder $R^3$ und $R^4$ beteiligt sind, seien z. B. cycloaliphatische $C_4$- bis $C_8$-Ringe genannt. Der Kohlenwasserstoffrest $R^4$ hat z. B. 1 bis 12 C-Atome. Er kann durch Halogenatome, Hydroxyl-, Keto-, Carbalkoxy-, Carboxamid-, Nitril- oder Acylgruppen substituiert sein. Kohlenwasserstoffreste der genannten Art sind z. B. Alkyl-, Cycloalkyl-, Alkenyl-, Alkinyl-, Aryl- oder Benzylgruppen.

Als cyclische Ether oder Lactonringe, an denen die Glieder $—OR^5$ und $R^4$ beteiligt sind, seien z. B.

gesättigte oder ungesättigte 4- bis 10-gliedrige, vorzugsweise 4- bis 6-gliedrige Ringe genannt.

Die als Ausgangsstoffe zu verwendenden Trichlormethylverbindungen können nach bekannten Methoden, z. B. durch säurekatalysierte Addition von Chloral an Olefine, durch Aldolreaktion von Chloral mit Carbonylverbindungen oder durch Addition von Trichlormethylanionen an Carbonylverbindungen erhalten werden.

In den nach dem erfindungsgemäßen Verfahren erhältlichen Chlorolefinen der allgemeinen Formel I sind die Reste $R^1$ und $R^2$ in der Mehrzahl der Fälle mit den Resten $R^3$ und $R^4$ der Ausgangsverbindungen der Formel II identisch. $R^1$ und $R^2$ können von $R^3$ und $R^4$ aber auch verschieden sein. So erhält man z. B. aus der Ausgangsverbindung der Formel II, in der $R^3$ und $R^5$ Wasserstoffatome und $R^4$ eine Nitrilgruppe bedeuten, ein Chlorolefin der Formel I, in der $R^1$ ein Wasserstoffatom bedeutet und $R^2$ für eine Carboxamidgruppe steht.

Die Erfindung betrifft ferner die neuen Chlorolefine der allgemeinen Formel

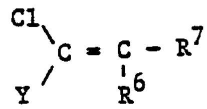 (III)

in der Y ein Chlor- oder Wasserstoffatom, $R^6$ ein Wasserstoffatom oder eine Methylgruppe, $R^7$ einen der Reste

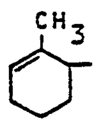, 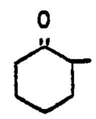, 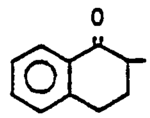, 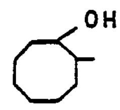, 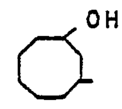, 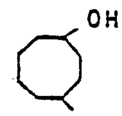,

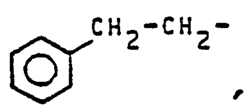, 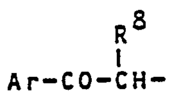 oder 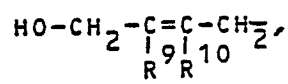,

Ar einen Arylrest, $R^8$ eine Methylgruppe oder ein Wasserstoff-, Chlor- oder Bromatom, und die Reste $R^9$ und $R^{10}$ je ein Wasserstoffatom oder eine Methylgruppe bedeuten.

Diese neuen Chlorolefine können z. B. als Zwischenprodukte für die Herstellung von Pharmaka und Pflanzenschutzmitteln verwendet werden.

Nach dem erfindungsgemäßen Verfahren behandelt man die Ausgangsverbindungen in wäßrigem Medium mit den Chrom-II-salzen. Besonders geeignet sind Chrom-II-salze von Mineralsäuren oder Carbonsäuren, insbesondere die Chloride, Sulfate und Acetate, von denen die Chloride oder Acetate bevorzugt sind. Das Chrom liegt in diesen Salzen meist in Form einer Komplexverbindung vor, die z. B. Aquo- oder Amino-Liganden aufweist. Die Chromsalze werden vorzugsweise in wäßriger Lösung eingesetzt. Die wäßrige Lösung sollte einen pH-Wert von $< 5$, insbesondere $\leq 3{,}5$, aufweisen, um die Hydrolyse der Chromsalze zu unterbinden. Man verwendet zweckmäßigerweise die 2- bis 6-fache molare Menge an Chrom-II-salz, bezogen auf ein Mol Trichlormethylverbindung.

Man behandelt die Trichlormethylverbindungen mit den Chrom-II-salzen bei Temperaturen von - 35 bis 110 °C, vorzugsweise 0 bis 50 °C und bei Drücken von 0 bis 20, vorzugsweise 0,7 bis 2 bar. Der Gehalt an $Cr^{2+}$ im wäßrigen Umsetzungsgemisch liegt z. B. zwischen 3 und 0,001 Mol/kg. Die Anfangskonzentration an $Cr^{2+}$ liegt zweckmäßig bei 0,01 Mol bis 2 Mol/kg. Das Reaktionsmedium kann rein wäßrig sein, es kann aber auch aus einem Gemisch aus Wasser und einem organischen Lösungsmittel bestehen, wobei der Gehalt an Lösungsmitteln bis zu 70 Gew.% betragen kann. Der Lösungsmittelgehalt geht vorzugsweise über 50 Gew.% nicht hinaus. Geeignete Lösungsmittel sind besondere mit Wasser mischbare organische Lösungsmittel, wie Dimethylformamid, Tetrahydrofuran, Acetonitril oder Dioxan. Man kann aber auch zweiphasig arbeiten und mit Wasser nicht mischbare Lösungsmittel, wie Toluol, Diethylether oder Methylenchlorid verwenden.

Bei einer besonders vorteilhaften Ausführungsform des Verfahrens der Erfindung verwendet man eine Lösung von Chrom-II-salzen, die durch elektrochemische Reduktion einer wäßrigen Lösung eines Chrom-III-salzes hergestellt wurde.

Dabei kann man so verfahren, daß man die durch Elektrolyse gewonnene wäßrige Chrom-II-salz-Lösung nach Entnahme aus der Elektrolysezelle für die Reduktion der Trichlormethylverbindungen einsetzt (« ex cell » Fahrweise). Man kann die erfindungsgemäße Reduktion aber auch gleichzeitig, d. h. während der Elektrolyse im Kathodenraum der Elektrolysezelle durchführen (« in cell » Fahrweise), wobei die Chromsalze fortlaufend regeneriert werden.

Man verwendet für die Elektrolyse z. B. an sich bekannte geteilte Zellen, insbesondere von Ionenaustauschermembranen unterteilte Platten- und Rahmenzellen. Als Kathodenmaterial werden die

üblichen Materialien, wie Quecksilber oder andere Metalle mit mittlerer bis hoher Wasserstoffüberspannung verwendet, insbesondere Blei, Zinn, Zink. Auch Graphit oder beschichtete Graphitsorten, wie sie in der DE-OS 33 00 865 beschrieben sind, sind geeignet. Bei der « in cell »-Fahrweise elektrolysiert man z. B. bei Temperaturen von 0 bis 80 °C und bei Stromdichten von 0,1 bis 30 Ampere/$dm^2$. Als Anolyt verwendet man z. B. verdünnte Schwefelsäure oder Salzsäure, wobei der Anolyt auch noch die obengenannten Chromsalze enthalten kann. Die Regenerierung der Chromsalze nach ihrem Einsatz bei der erfindungsgemäßen Reduktion gemäß der « ex cell »-Fahrweise läßt sich ebenfalls vorteilhaft durch Elektrolyse vornehmen.

Nach dem neuen Verfahren lassen sich die Trichlormethylverbindungen besonders vorteilhaft zu den entsprechenden Chlorolefinen reduzieren. Daß diese Reduktion in wäßrigem Medium so glatt verläuft, ist überraschend, da bei bisherigen Versuchen, α-Halogenalkohole mit Chrom-II-salzen zu reduzieren (s. Wellmann, Diss. « Indirekte elektrochemische Reduktionen organischer Verbindungen mit anorganischen Elektronenüberträgern », Münster, 1979) positive Ergebnisse nur in organischen Lösungsmitteln bei weitgehender Abwesenheit von Wasser und vor allem nur bei den stärker aktivierten Bromverbindungen erhalten wurden.

Aufgrund des überraschenden Befundes, daß sich das Verfahren dieser Erfindung so glatt in wäßrigem Medium durchführen läßt, lassen sich die erheblichen Vorteile einer Kombination des Verfahrens mit der Regenerierung der Chromsalze technisch wirtschaftlich nutzen. Als Anolyt können rein wäßrige Lösungen eingesetzt werden. Da im Katholyten eine vorwiegend wäßrige Phase verwendet wird, ist auch die Kontamination des Anolyten durch den Katholyten gering. Wie der Vergleichsversuch ohne Chromsalze zeigt (s. Beispiel 23), erhält man bei der direkten elektrochemischen Reduktion der Trichlormethylverbindungen in wäßrigem Milieu auch nach Zugabe von Lösungsvermittlern nur schlechte Ergebnisse.

## Beispiele 1 bis 13

1 Volumenteil einer 2-molaren wäßrigen Lösung von $CrCl_2$ wurde mit 1 Volumenteil Dimethylformamid unter Sauerstoffausschluß bei 0 °C vermischt. In dieses Gemisch wurde unter Rühren so viel der aus der Tabelle ersichtlichen Trichlormethylverbindung eingetragen, daß das $CrCl_2$ in 4,2-fachem molaren Überschuß vorlag. Der pH-Wert der Lösung betrug 3,5. Das Reaktionsgemisch wurde dann 2 bis 16 Stunden bei Raumtemperatur gerührt. Die Umsatzkontrolle erfolgte gaschromatographisch oder dünnschichtchromatographisch. Zur Aufarbeitung wurde zum Reaktionsgemisch das 3- bis 6-fache Volumen an Wasser gegeben. Dann wurde mehrmals mit Pentan extrahiert. Nach Waschen und Trocknen wurde das Lösungsmittel abdestilliert. Die als Rückstand erhaltene rohe Chlorolefinverbindung wurde zur Reinigung fraktioniert destilliert oder in einem Gemisch aus Methylenchlorid/Cyclohexan 1 : 0 bis 1 : 1 gelöst und chromatographiert.

Das verwendete $CrCl_2$ wurde in üblicher Weise durch Behandlung von $CrCl_3$ mit Zink oder durch elektrochemische Reduktion gemäß Beispiel 22, Absatz c, hergestellt.

(Siehe Tabellen Seite 5 ff.)

| Beispiel | Trichlormethylverbindung | Chlorolefin und Ausbeute in Gew.% | Dauer der Umsetzung [h] | Stereochemie der Doppelbindung (nach NMR) |
|---|---|---|---|---|
| 1 | 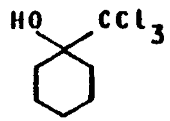 | 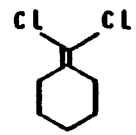 63 % 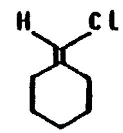 11 % | 2 | - |
| 2 | 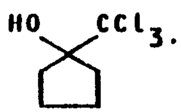 | 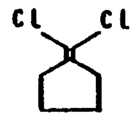 40 % 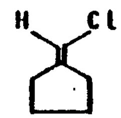 23 % | 15 | - |
| 3 | 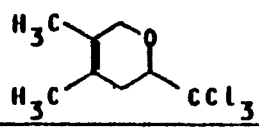 | $HO-CH_2-C(CH_3)=C(CH_3)-CH_2-CH=CHCl$ 66% | 3 | beide cis |
| 4 | 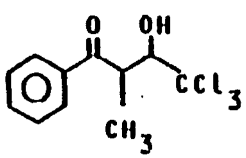 | 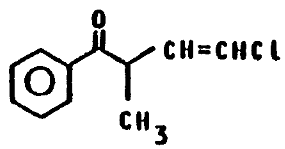 62 % | 2 | cis |
| 5 | 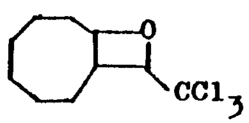 | 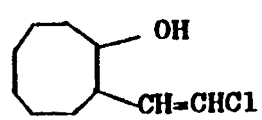 71 % 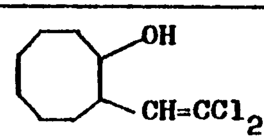 13 % | 4 | cis |

| Bei-spiel | Trichlormethylverbindung | Chlorolefin und Ausbeute in Gew.% | Dauer der Umsetzung [h] | Stereochemie der Doppelbindung (nach NMR) |
|---|---|---|---|---|
| 6 | 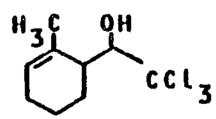 | 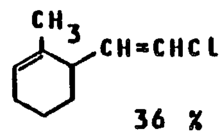 36 % | 4 | cis |
| 7 | 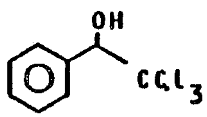 | 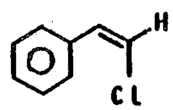 44 % 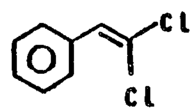 10 % | 3 | cis |
| 8 | 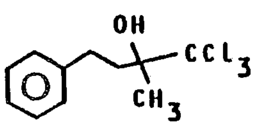 | 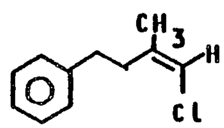 13 % 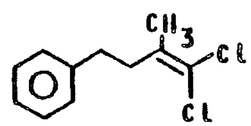 56 % | 16 | cis/trans-Gemisch |
| 9 | 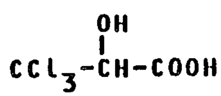 | 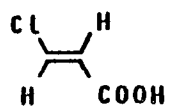 30 % | 5 | trans |

| Bei-spiel | Trichlormethylverbindung | Chlorolefin und Ausbeute in Gew.% | Dauer der Umsetzung [h] | Stereochemie der Doppelbindung (nach NMR) |
|---|---|---|---|---|
| 10 | 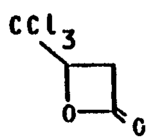 | 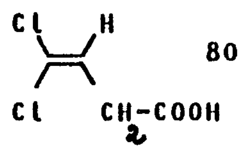 80 % | 5 | - |
| 11 | 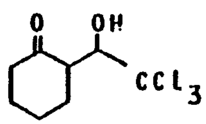 | 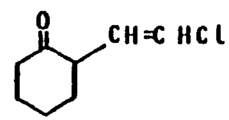 90 % | 2 | bevorzugt cis |
| 12 | $CCl_3-CH(OAc)_2$ | Cl-CH=CH-OAc 30 % 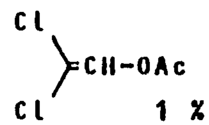 1 % | 16 | cis/trans 95:5 |
| 13 | 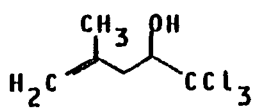 | 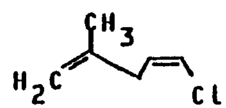 60 % | 6 | cis |

## Beispiele 14 und 15

Es wurden die Beispiele 4 und 5 mit einem anderen Mischungsverhältnis zwischen Wasser und Dimethylformamid (DMF) wiederholt.

| Beispiel | Trichlormethylverbindung | $H_2O$/DMF Gew.Verh. | Chlorolefin und Ausbeute in Gew.% | |
|---|---|---|---|---|
| 14 | s. Beispiel 4 | 75/25 | 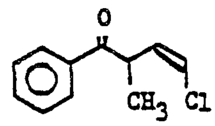 | 60 % |
| 15 | s. Beispiel 5 | 85/15 | 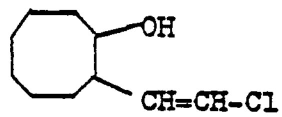 | 13 % |
| | | | 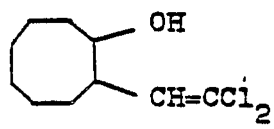 | 64 % |

## Beispiel 16

a) Herstellung der Trichlormethylverbindung

In einem 2-Hals-Kolben, ausgestattet mit Magnetrührer, Innenthermometer und Trockenrohr wurden 44,10 g (0,4 mol) Cycloocten und 58,95 g (0,4 mol) Chloral in 60 ml Petrolether vorgelegt. Durch ein Kältebad wurde der Ansatz auf ≤ 0 °C gekühlt. Innerhalb von 15 min gab man portionsweise 5.5 g (0.04 mol) Aluminiumtrichlorid zu, wobei man die Temperatur auf 0 °C hielt. Man rührte noch weitere 4 h bei 0 °C und ließ anschließend 15 h bei Raumtemperatur stehen. Zur Hydrolyse wurde auf 0 °C gekühlt und langsam mit dem gleichen Volumen Eiswasser versetzt. Die dunkelbraune Petroletherphase wurde abgetrennt, zur Entfernung unlöslicher Polymerer filtriert und 3 mal mit gesättigter wäßriger Natriumchloridlösung gewaschen. Die organische Phase wurde über Magnesiumsulfat getrocknet und am Rotationsverdampfer eingeengt. Zur Reinigung wurde das Rohprodukt im Vakuum fraktioniert destilliert (105 bis 118 °C ; 0,5 Torr). Man erhielt 78 g (0.3 mol ; 75 Gew.%) eines Gemisches zweier Verbindungen im Verhältnis 1 : 1. Die Trennung der beiden Verbindungen erfolgte durch Flüssigkeitschromatographie (Säule : Ø = 5 cm ; Länge = 20 cm) auf einer Kieselgelsäule mit dem Laufmittel Cyclohexan/Methylenchlorid (1 : 1). Man isolierte neben 3-(1'-Hydroxy-2',2',2'-trichlorethyl)-1-cycloocten eine Verbindung der vermutlichen Struktur 8-Trichlormethyl-7-oxa-bicyclo [4.2.2]-decan der Formel

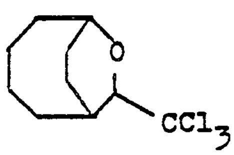

b) Herstellung der Chlorolefinverbindung

In einem Rührkolben wurden 9 ml Dimethylformamid (DMF) vorgelegt und mit einem Argonstrom von Sauerstoff befreit. Anschließend wurden unter Eisbadkühlung langsam 9.0 ml einer wäßrigen 1.48-molaren (13.4 mmol) Cr(II)-chloridlösung vom pH 3.5 zugefügt. Danach gab man 0.86 g (3.3 mmol) der Trichlormethylverbindung mit der oben angegebenen vermutlichen Struktur in wenig DMF gelöst hinzu. Das Eisbad wurde entfernt und man rührte noch 5 h. Zur Aufarbeitung wurde die Lösung mit dem 4-fachen Volumen Wasser versetzt und mit Pentan extrahiert. Die organische Phase wurde gewaschen und über Magnesiumsulfat getrocknet. Das Lösungsmittel entfernte man destillativ. Das Gaschromatogramm zeigte auf einer UV 101-Säule zwei intensive Peaks für das Produkt (rel. Peakfläche 65 %) und das unumgesetzte Edukt (15 % rel. Peakfläche). Zur präparativen Trennung wurde das Rohprodukt (0.78 g) auf eine Kieselgelsäule mit Methylenchlorid als Laufmittel getrennt. Man erhielt so 0.42 g (67 % auf

Einsatz ; 71 % auf Umsatz) des Produkts mit der vermutlichen Struktur : (Z)-4-(2'-chlorethen-1'-yl)-cyclooctan-1-ol der Formel

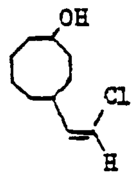

## Beispiele 17 bis 21

Es wurde wie in den Beispielen 1 bis 13 angegeben verfahren, wobei jedoch kein Dimethylformamid und auch kein anderes organisches Lösungsmittel verwendet wurde. Der pH-Wert der Lösungen betrug bei den Beispielen 17 und 18 3.5. Bei den Beispielen 19, 20 auf 21 wurden die Lösungen durch Zugabe von HCl auf einen pH-Wert von 1 gestellt.

| Beispiel | Trichlormethylverbindung | Chlorolefin und Ausbeute in Gew.% | |
|---|---|---|---|
| 17 | 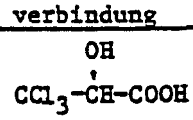 | 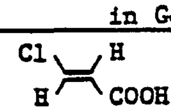 | 30 % |
| 18 | 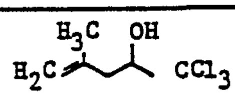 | 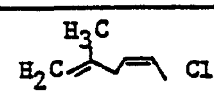 | 55 % |
| 19 | 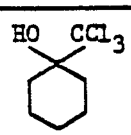 | 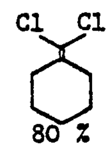 80 % 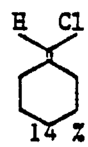 14 % | |
| 20 | 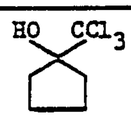 | 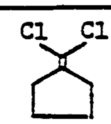 77 % 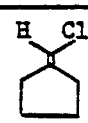 8 % | |
| 21 | 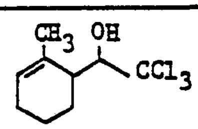 | 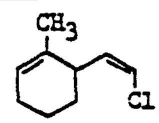 | 8 % |

## Beispiel 22

Kombination der erfindungsgemäßen Reduktion mit der elektrolytischen Gewinnung von $CrCl_2$

a) Elektrolyse

Die Elektrolyse wurde in einer geteilten Zelle mit einer $Pb/PbO_2$ Anode der Oberfläche 1 $dm^2$ und einer Pb-Kathode der Oberfläche 1 $dm^2$ durchgeführt. Anodenraum und Kathodenraum waren durch eine ®NAFION-Kationenaustauschermembran voneinander getrennt. Als Anolyt wurde 1 kg 5 %ige Schwefelsäure und als Katholyt eine Lösung von 106,5 g $CrCl_3 \cdot 6H_2O$ in 750 ml Wasser verwendet.

b) « In-Cell »-Reduktion

Dem Katholyten wurde eine Lösung von 18,9 g (0,1 Mol) 3-Trichlormethylpropiolacton in 500 ml Tetrahydrofuran zugegeben. Bei Raumtemperatur wurde 3 Stunden bei einer Stromstärke von 5 A elektrolysiert. Dann wurde der Katholyt mit Methylenchlorid extrahiert. Die organische Phase wurde

getrocknet und im Rotationsverdampfer eingedampft. Es wurden 13,2 g (4,4-Dichlor)-3-butensäure (NMR-spektroskopisch rein) erhalten. Ausbeute : 85 Gew.%.

c) « Ex-Cell »-Reduktion

Zunächst wurde die in Absatz a) beschriebene $CrCl_3$-Lösung gemäß Absatz b) bei einer Stromstärke von 5 A elektrolysiert. Die so gewonnene $CrCl_2$-Lösung wurde zu einer Lösung von 18,9 g (0,1 Mol) 3-Trichlormethylpropiolacton in 500 ml Tetrahydrofuran gegeben. Das Gemisch wurde 2 Stunden bei Raumtemperatur gerührt. Das Reaktionsgemisch wurde wie in Absatz (b) beschrieben aufgearbeitet. Es wurden 11,9 g (4,4-Dichlor)-3-butensäure (NMR-spektroskopisch rein) erhalten. Ausbeute : 77 Gew.%.

d) Wiederverwendung der Chromsalzlösung aus c)

Die bei der Ex-Cell-Reduktion gemäß Absatz c) zurückgewonnene wäßrige Chromsalzlösung wurde erneut für eine Elektrolyse nach Absatz c) verwendet. Bei der anschließenden Umsetzung mit 3-Trichlormethylpropiolacton wurde wie in Absatz c) beschrieben verfahren, wobei jedoch anstelle von 500 ml Tetrahydrofuran 300 ml Dimethylformamid verwendet wurden. Man erhielt 20,2 g Rohprodukt (Gehalt an (4,4-Dichlor)-3-butensäure etwa 15 g, Rest Dimethylformamid). Ausbeute : etwa 97 Gew.%. Nach Destillation ($Kp_2$ : 95 °C) ergab die Analyse folgende Werte :

| | | | | |
|---|---|---|---|---|
| gef. | C 31,6 % | H 2,9 % | O 20,9 % | Cl 45,4 % |
| ber. | C 30,9 % | H 2,6 % | O 20,7 % | Cl 45,8 % |

e) « In-Cell »-Reduktion unter Wiederverwendung des Elektrolyten

Dem Katholyten wurde eine Lösung von 189,5 g (1 Mol) 3-Trichlormethylpropiolacton in 500 ml Tetrahydrofuran (THF) zugesetzt. Es wurde 7,75 h lang bei 50 °C und einer Stromstärke von 8 A elektrolysiert (Stromeinsatz 62 Ah = 116 % d. Th.). Anschließend wurde der Katholyt durch dreifaches Extrahieren mit je 200 ml Methylenchlorid vom Wert-produkt befreit. Danach wurde er erneut mit 3-Trichlormethylpropiolacton in Tetrahydrofuran versetzt und der Elektrolyse zugeführt. Dieser Vorgang wurde dreimal wiederholt.

Die organische Phase wurde mit wenig Wasser gewaschen, getrocknet und im Rotationsverdampfer eingedampft. Die Rohausbeute betrug > 95 %. Zur Reingewinnung des Wertproduktes wurde das Rohprodukt (150,3 g gelbes Öl = 97 %) mit 300 g Wasser 1/2 h ausgerührt. Die entstandene Kristallmaische wurde abgekühlt und abgesaugt. Der Niederschlag wurde getrocknet. Es wurden 129,3 g (84 %) (4,4-Dichlor)-3-butensäure in Form weißer Kristalle vom Schmelzpunkt 42 °C erhalten.

Beispiele 23 (Vergleichsversuch)

Die im Beispiel 18, Absatz b), beschriebene Elektrolyse wurde wiederholt, wobei jedoch als Katholyt anstelle der Chromsalzlösung eine 5 gew.%ige wäßrige Kochsalzlösung verwendet wurde. Bei der Aufarbeitung wurden 18,2 g eines Gemisches erhalten, das zu etwa 50 Gew.% aus der Ausgangsverbindung, zu etwa 40 Gew.% aus 4,4,4-Trichlor-3-hydroxi-butansäure und zu etwa 10 Gew.% aus (4,4-Dichlor)-3-butensäure bestand.

**Patentansprüche**

1. Verfahren zur Herstellung von Chlorolefinen der Formel

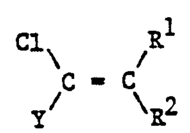

. (I)

in der Y ein Chlor- oder Wasserstoffatom, $R^1$ ein Wasserstoffatom, einen Kohlenwasserstoffrest oder einen Rest, der zusammen mit $R^2$ Glied eines Kohlenwasserstoffringes ist, $R^2$ eine Carboxyl-, Carbalkoxy-, Carboxamid-, Nitril- oder Acylgruppe, einen Kohlenwasserstoffrest, der Halogenatome, Hydroxyl-, Keto-, Carboxyl-, Carbalkoxy-, Carboxamid-, Nitril- oder Acylgruppen enthalten kann, oder einen Rest, der zusammen mit $R^1$ Glied eines Kohlenwasserstoffringes ist, bedeuten, dadurch gekennzeichnet, daß man Trichlormethylverbindungen der Formel

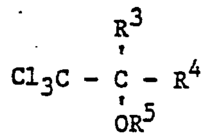

(II)

in der $R^3$ ein Wasserstoffatom, einen Kohlenwasserstoffrest oder einen Rest, der zusammen mit $R^4$ Glied eines Kohlenwasserstoffringes ist, $R^4$ eine Carboxyl-, Carbalkoxy-, Carboxamid-, Nitril- oder Acylgruppe, einen Kohlenwasserstoffrest, der Halogenatome, Hydroxyl-, Keto-, Carboxyl-, Carbalkoxy-, Carboxamid-, Nitril- oder Acylgruppen enthalten kann, oder einen Rest, der zusammen mit $R^3$ Glied eines Kohlenwasserstoffringes ist, oder einen Rest, der zusammen mit $-OR^5$ Glied eines cyclischen Ethers oder eines Lactonringes ist, $R^5$ ein Wasserstoffatom, einen Alkyl- oder Acylrest oder einen Rest, der zusammen mit $R^4$ Glied eines cyclischen Ethers oder eines Lactonringes ist, bedeuten, in wäßrigem Medium mit Chrom-II-salzen behandelt.

2. Verfahren nach Anspruch 1, dadurch gekennzeichnet, daß man eine Lösung von Chrom-II-salzen verwendet, die durch elektrochemische Reduktion einer wäßrigen Lösung eines Chrom-III-salzes hergestellt wurde.

3. Verfahren nach Anspruch 2, dadurch gekennzeichnet, daß man die Behandlung der Trichlormethylverbindung mit den Chrom-II-salzen zusammen mit der elektrochemischen Reduktion der Chrom-III-salze im Kathodenraum der Elektrolysezelle durchführt.

4. Chlorolefine der allgemeinen Formel

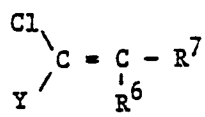

(III)

in der Y ein Chlor- oder Wasserstoffatom, $R^6$ ein Wasserstoffatom oder eine Methylgruppe, $R^7$ einen der Reste

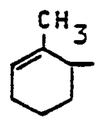 , 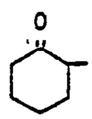 , 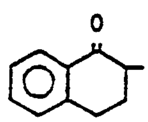 , 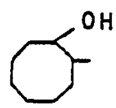 , 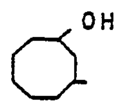 , 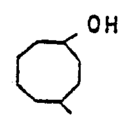

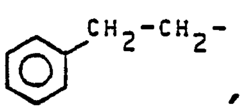 , 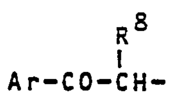 oder 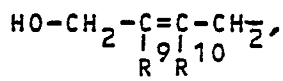 ,

Ar einen Arylrest, $R^8$ eine Methylgruppe oder ein Wasserstoff-, Chlor- oder Bromatom, und die Reste $R^9$ und $R^{10}$ je ein Wasserstoffatom oder eine Methylgruppe bedeuten.

**Claims**

1. A process for the preparation of a chloro-olefin of the formula

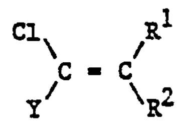

(I)

where Y is chlorine or hydrogen, $R^1$ is hydrogen, a hydrocarbon radical, or a radical which, together with $R^2$, is a member of a hydrocarbon ring, $R^2$ is a carboxyl, carbalkoxy, carboxamide, nitrile or acyl group, a hydrocarbon radical which may contain halogen atoms or hydroxyl, keto, carboxyl, carbalkoxy, carboxamide, nitrile or acyl groups, or a radical which, together with $R^1$, is a member of a hydrocarbon ring, wherein a trichloromethyl compound of the formula

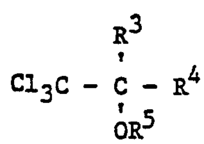

(II)

where $R^3$ is hydrogen, a hydrocarbon radical, or a radical which, together with $R^4$, is a member of a hydrocarbon ring, $R^4$ is a carboxyl, carbalkoxy, carboxamide, nitrile or acyl group, a hydrocarbon radical which may contain halogen atoms or hydroxyl, keto, carboxyl, carbalkoxy, carboxamide, nitrile or acyl groups, or a radical which, together with $R^3$, is a member of a hydrocarbon ring, or a radical which,

together with —$OR^5$, is a member of a cyclic ether or of a lactone ring, and $R^5$ is hydrogen, alkyl, acyl or a radical which, together with $R^4$, is a member of a cyclic ether or of a lactone ring, is treated with a chromium (II) salt in an aqueous medium.

2. A process as claimed in claim 1, wherein the solution of the chromium (II) salt used has been prepared by electrochemical reduction of an aqueous solution of a chromium (III) salt.

3. A process as claimed in claim 2, wherein the treatment of the trichloromethyl compound with the chromium (II) salt is carried out together with the electrochemical reduction of the chromium (III) salt in the cathode chamber of the electrolysis cell.

4. A chloro-olefin of the general formula

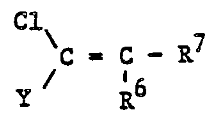

(III)

where Y is chlorine or hydrogen, $R^6$ is hydrogen or methyl, $R^7$ is one of the radicals

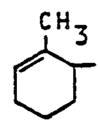 , 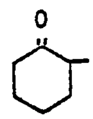 , 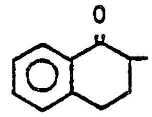 , 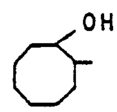 , 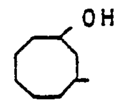 , 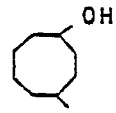

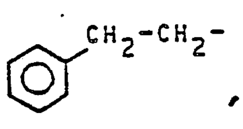 , 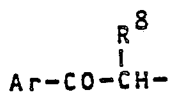 oder 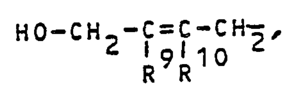 ,

Ar is aryl, $R^8$ is methyl, hydrogen, chlorine or bromine, and $R^9$ and $R^{10}$ are each hydrogen or methyl.

**Revendications**

1. Procédé pour la préparation d'oléfines chlorées de formule

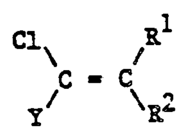

(I)

dans laquelle Y représente un atome de chlore ou d'hydrogène, $R^1$ représente un atome d'hydrogène, un radical hydrocarboné ou un radical qui forme, avec $R^2$, un maillon d'un noyau d'hydrocarbure et $R^2$ représente un groupe carboxyle, carbalcoxy, carboxamide, nitrile ou acyle, un radical hydrocarboné qui peut contenir des atomes d'halogène, des groupes hydroxyle, céto, carboxyle, carbalcoxy, carboxamide, nitrile ou acyle, ou un radical qui forme, avec $R^1$, un maillon d'un noyau d'hydrocarbure, caractérisé en ce qu'on traite en milieu aqueux, par des sels chromeux, des composés trichlorométhylés de formule

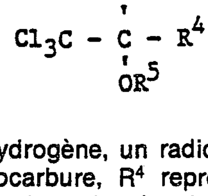

(II)

dans laquelle $R^3$ représente un atome d'hydrogène, un radical hydrocarboné ou un radical qui forme, avec $R^4$, un maillon d'un noyau d'hydrocarbure, $R^4$ représente un groupe carboxyle, carbalcoxy, carboxamide, nitrile ou acyle, un radical hydrocarboné qui peut contenir des atomes d'halogène, des groupes hydroxyle, céto, carboxyle, carbalcoxy, carboxamide, nitrile ou acyle, un radical qui forme, avec $R^3$, un maillon d'un noyau d'hydrocarbure ou un radical qui forme, avec —$OR^5$, un maillon d'un éther cyclique ou d'un noyau lactonique, $R^5$ représente un atome d'hydrogène, un radical alkyle ou acyle ou un radical qui forme, avec $R^4$, un maillon d'un éther cyclique ou d'un noyau lactonique.

2. Procédé selon la revendication 1, caractérisé en ce qu'on utilise une solution de sels chromeux qui a été préparée par réduction électrochimique d'une solution aqueuse d'un sel chromique.

3. Procédé selon la revendication 2, caractérisé en ce qu'on effectue le traitement du composé

trichlorométhylé par les sels chromeux en même temps que la réduction électrochimique des sels chromiques dans la région cathodique de la cellule d'électrolyse.

4. Oléfines chlorées de formule générale

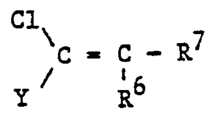

(III)

dans laquelle Y représente un atome de chlore ou d'hydrogène, $R^6$ représente un atome d'hydrogène ou un groupe méthyle, $R^7$ représente l'un des radicaux

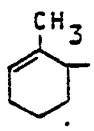 , 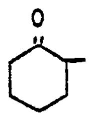 , 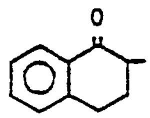 , 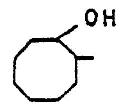 , 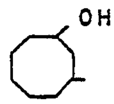 , 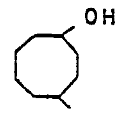

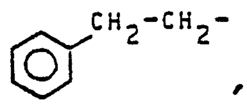 , 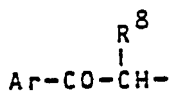 oder 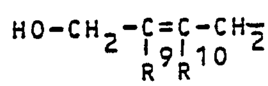,

Ar représentant un radical aryle, $R^8$ représentant un groupe méthyle ou un atome d'hydrogène, de chlore ou de brome et les radicaux $R^9$ et $R^{10}$ représentant chacun un atome d'hydrogène ou un groupe méthyle.